# EUROPEAN PATENT SPECIFICATION

(11) **EP 2 462 130 B1**
(45) Date of publication and mention of the grant of the patent: **03.07.2013**
(21) Application number: 10742065.5
(22) Date of filing: 04.08.2010
(51) Int. Cl.: C07D 301/12

(54) **PROCESS FOR PRODUCING PROPYLENE OXIDE**
VERFAHREN ZUR HERSTELLUNG VON PROPYLENOXIDE
PROCÉDÉ DE PRÉPARATION DE L' OXYDE DE PROPYLÈNE

(30) Priority: 05.08.2009 US 231416 P
(43) Date of publication of application: 13.06.2012
(73) Proprietor: Dow Global Technologies LLC, Midland, MI 48674 (US)
(72) Inventor: CRAMPTON, Hannah, L., Lake Jackson TX 77566 (US); CARLBERG, Philip, J., Lake Jackson TX 77566 (US); WEST, David, H., Houston TX 77041 (US); HOOK, Bruce, D., Lake Jackson TX 77566 (US); FAN, William, W., Lake Jackson TX 77566 (US); FORLIN, Anna, I-35010 Vigonza (IT)
(74) Representative: Raynor, John
(86) International application number: PCT/US2010/044355
(87) International publication number: WO 2011/017402

(56) References cited:
- US-B2- 6 372 924
- ZHAOGUANG ZHANG ET AL: "Effects of organic solvent addition on the epoxidation of propene catalyzed by TS-1", REACTION KINETICS AND CATALYSIS LETTERS, SPRINGER SCIENCE+BUSINESS MEDIA, DORDRECHT, NL, vol. 92, no. 1, 21 September 2007 (2007-09-21), pages 49-54, XP019533263, ISSN: 1588-2837, DOI: DOI:10.1007/S11144-007-5092-6 cited in the application
- KAWABATA TOMONORI ET AL: "Liquid-phase epoxidation of propylene with hydrogen peroxide using Ti-MWW catalyst", STUDIES IN SURFACE SCIENCE AND CATALYSIS, ELSEVIER BV, NL, vol. 172, 1 January 2007 (2007-01-01), pages 551-552, XP009141922, ISSN: 0167-2991
- YOSHINAO NAKAGAWA, KEIGO KAMATA, MIYUKI KOTANI, KAZUYA YAMAGUCHI AND NORITAKA MIZUNO: "Polyoxovanadometalate-Catalysed Selective Epoxidation of Alkenes with Hydrogen Peroxide", ANGEW. CHEM. INT. ED., vol. 44, 2005, - 2005, pages 5136-5141, XP002613508, DOI: 10.102/anie.200500491
- WEIGUO CHENG, XIANGSHENG WANG, GANG LI, XINWEN GUO AND BINGJUAN DU: "Characterization and catalytic activity of hydrothermally treated TS-1/SiO2 in propylene epoxidation", CATALYSIS LETTERS, vol. 95, no. 3-4, June 2004 (2004-06), - June 2004 (2004-06), pages 185-190, XP002613509,
- CLERICI, M. G. ET AL: "Synthesis of propylene oxide from propylene and hydrogen peroxide catalyzed by titanium silicalite", JOURNAL OF CATALYSIS , 129(1), 159-67 CODEN: JCTLA5; ISSN: 0021-9517, 1991, XP009141906,

## Description

### BACKGROUND OF THE INVENTION

### Field of the Invention

The present invention relates to a process for preparing propylene oxide by reacting propylene and a peroxide compound, in the presence of a catalyst such as a titanium silicalite-1 (TS-1) catalyst and in the presence of a mixed solvent system.

### Background of the Invention and Related Art

In the preparation of propylene oxide, the customary processes in the prior art involve reacting propylene with hydrogen peroxide to produce propylene oxide. The process is usually carried out in one or more stages. For example, U.S. Patent Nos. 6,479,680; 6,849,162; 6,867,312; 6,881,853; 6,884,898; 6,960,671; 7,173,143; 7,332,634; 7,378,536; and 7,449,590; disclose a process for the preparation of propylene oxide using hydrogen peroxide in the presence of a catalyst such as a titanium silicalite-1 (TS-1) catalyst, and in the presence of a solvent such as methanol.

A problem with propylene oxide production using the above prior art processes, is that the activity of the catalyst deteriorates with the passage of time. Typically, TS-1 catalyst, used under the process conditions of the prior art, deactivates in a matter of hours, with full regeneration attainable only by calcination.

Another problem with the prior art propylene oxide production processes relates to the use of methanol as a solvent for the processes. While methanol is a necessary component of the peroxide reaction to obtain high activity, the methanol is generally used in large excesses. For example, Clerici et al., "Epoxidation of Lower Olefins with Hydrogen Peroxide and Titanium Silicalite," Journal of Catalysis, 1993, 140, 71-83, discloses a process that is representative of TS-1/H₂O₂ epoxidation processes, wherein the methanol level used in an example is approximately 97%. The use of an excessive amount of methanol results in the formation of a single phase during the reaction, but suffers from the formation of byproducts from the reaction of methanol and water, which is solubilized in the organic phase by methanol, with propylene oxide. The use of large quantities of methanol also results in the need for large towers for propylene oxide production facilities, and for a high energy consumption for the purification of the product produced on a commercial scale.

In an attempt to increase the selectivity of a catalyst, other prior art processes involve the addition of further selectivity enhancing compounds added to the reaction mixture. For example, U.S. Patent No. 7,323,578 discloses that both a continuous and a batch process for the production of an oxirane by the reaction of an olefin and a peroxide compound can be used and that the liquid phase of the reaction contains water, one or more organic solvents, a catalyst, and one or more compounds that increase the selectivity of the catalyst for epoxidation reactions. The compound for increasing the selectivity is chosen from inorganic or organic bases, salts and their conjugate acid or base, salts, or mixtures of any of the previous. However, U.S. Patent No. 7,323,578 only teaches process conditions that result in a single liquid phase; and that adding an additional component is necessary for increasing the selectivity towards oxirane and for lengthening the lifetime of the catalyst.

Pandey et al., "Eco-friendly Synthesis of Epichlorohydrin Catalyzed by Titanium Silicalite (TS-1) Molecular Sieve and Hydrogen Peroxide," Catalysis Communications, 2007, 8, 379-382, describes reaction conditions for making an oxide with TS-1/H₂O₂ using a solvent mixture of methanol and acetonitrile. However, in the process disclosed in the above publication, the total solvent amount used creates single phase reaction conditions.

Zhang et al., "Effects of Organic Solvent Addition on the Epoxidation of Propene Catalyzed by TS-1," Reaction Kinetics and Catalysis Letters, 2007, 92(1), 49-54, discusses the use of solvent mixtures with methanol for the epoxidation of propylene. Zhang et al. disclose that replacing -24% of the methanol in Zhang et al.'s system with other solvents such as CCl₄, toluene, or 1, 2-dichloroethane resulted in increased selectivity and less clogging of the catalyst pores, as measured by thermogravimetric and pore volume analyses. The mixture of methanol and 1, 2-dichloroethane, which gives a total methanol composition of 60%, inhibits the decomposition of H₂O₂, and inhibits the reaction of propylene oxide into propylene glycol and propylene glycol mono-methyl ethers while retaining the H₂O₂ conversion as compared to methanol only. Although Zhang et al. describe using mixtures of solvents to increase the selectivity of the reaction, the total solvent amounts used create single phase reaction conditions.

In summary, the disadvantages of the known processes described in the above prior art include the following:
(1) A high level of methanol is used in the reactor of prior art processes, and thus, the high levels of methanol must be separated from the product and recycled. This creates a high energy use for the process and associated high costs.
(2) The prior art processes use a first separation step wherein solvent and unreacted reactant are recovered and recycled. This requires the use of a high temperature in the bottoms of distillations towers, which in turn requires the use of high temperatures throughout the distillation towers. Any water in the feed to the distillation towers remains in contact with the product in the distillation towers; and thus the available water provides an opportunity to react with the product to form undesired by-products.

Prior art processes which use solvent mixtures still use a high level of methanol, and/or still use a single liquid phase.

It is therefore desired to provide a process that does not have the problems of the above prior art processes and that can be operated at reaction conditions which address all of the above issues and maintain a high catalyst activity. In addition, it would be desirable to provide a propylene oxide production process which uses a low amount of total organic solvent (for example from 230-500 g/kg), and a much lower amount of methanol (for example, less than 100 g/kg) than previously known processes. Furthermore, it would be desirable to provide a process which would both increase the selectivity of the reaction and extend the lifetime of the catalyst without the need for any additional components, which would have to be removed in a subsequent downstream process.

### SUMMARY OF THE INVENTION

The present invention is directed to an improved process for the production of propylene oxide from propylene and hydrogen peroxide catalyzed by, for example, titanium silicalite-1 (TS-1), in a mixed solvent system.

One aspect of the present invention is directed to a multiple liquid phase composition, useful for preparing propylene oxide, comprising a reactive mixture of: (a) propylene, (b) at least one peroxide compound, (c) at least one catalyst, and (d) a solvent mixture; wherein the solvent mixture comprises at least (i) at least one alcohol or a combination of alcohols, and (ii) at least one non-reactive co-solvent other than the at least one alcohol, wherein the solvents are mixed at a predetermined concentration as defined in claim 1; wherein the non-reactive co-solvent has a different boiling point than the propylene oxide; and wherein the propylene oxide is capable of partitioning into at least one of the solvents present in the solvent mixture during the reaction.

Another aspect of the present invention is directed to a process for preparing propylene oxide from propylene and a peroxide compound including reacting (a) propylene with (b) at least one peroxide compound, in the presence of (c) at least one catalyst and (d) in the presence of a solvent mixture; wherein the solvent mixture comprises (i) at least one alcohol or a combination of alcohols at a predetermined concentration as defined in claim 10 and (ii) at least one non-reactive co-solvent at a predetermined concentration as defined in claim 10; wherein the non-reactive co-solvent has a different boiling point than the propylene oxide; and wherein the propylene oxide is capable of partitioning into at least one of the solvents present in the solvent mixture during the reaction.

The present invention is advantaged because it specifies a multiphase solvent system in which the propylene oxide produced is preferentially sequestered into the organic phase, reducing its contact with water and thus reducing byproducts resulting from solvolysis of the propylene oxide. This both increases the selectivity of the reaction and extends the lifetime of the catalyst. The multiphasic nature of the reaction mixture also facilitates the subsequent recovery of propylene oxide by allowing a simple separation of the aqueous and organic phases by, for example, decantation, prior to recovery of recyclable and useful product components from each stream.

In addition, the present invention is advantaged because the methanol concentrations presented in the present invention are lower than those presented in previously known processes, which decreases losses of the oxirane group of the propylene oxide to solvolysis by methanol, therefore increasing the selectivity and maximizing peroxide use.

It has been found that while the present reaction is possible without the presence of methanol or solvent, the catalyst lifetime may be extremely short and may only be regenerated through calcination. The present invention is advantaged because, while the present invention specifies a multiphase solvent system, the presence of a small amount of methanol, along with a non-reacting cosolvent, causes the propylene oxide produced to be preferentially sequestered into the organic phase, reducing its contact with water and thus reducing by products resulting from solvolysis of the oxirane group of the propylene oxide. This both increases the selectivity of the reaction and extends the lifetime of the catalyst.

### BRIEF DESCRIPTION OF THE DRAWINGS

The following drawings illustrate non-limiting embodiments of the present invention, wherein:
Figure 1 is a schematic flow diagram showing the overall flows in and out of a reaction/phase separation unit operation for one embodiment of the process of the present invention;
Figure 2 is a schematic flow diagram showing a reaction unit operation separate from a phase separation unit operation wherein the reaction/phase separation is separate from the product and recycle stream separation section for one embodiment of the process of the present invention; and
Figure 3 is a schematic flow diagram showing a reaction/phase separation unit operation and four blocks of unit operations to separate products from byproducts and unreacted reactants following the reaction/phase separation for one embodiment of the process of the present invention.
Figure 4 is a schematic flow diagram of an embodiment of the present invention showing two reactors in series with counter-current flows.

### DETAILED DESCRIPTION OF THE INVENTION

In its broadest scope, the present invention relates to preparing propylene oxide, comprising reacting (a) propylene with (b) at least one peroxide compound, in the presence of (c) at least one catalyst, and (d) in the presence of a solvent mixture; wherein the solvent mixture comprises at least (i) at least one or more alcohols at a predetermined concentration, and (ii) at least one non-reacting co-solvent at a predetermined concentration.

The olefin useful in the process of the present invention comprises propylene; and a preferred embodiment of the present invention includes a process wherein propylene is epoxidized to propylene oxide using aqueous hydrogen peroxide and a catalyst such as titanium silicalite catalyst, TS-1.

The concentration of the propylene is generally between about 1 weight percent (wt%) to about 90 wt%, preferably between about 4 wt% to about 50 wt%, more preferably between about 4 wt% to about 30 wt%, and most preferably between about 4 wt% to about 20 wt%, based on the total weight of the composition which includes all of the components fed to a reactor to form the reaction mixture including for example the weight all of the liquid components and the catalyst together herein "the total composition." In one preferred embodiment of the present invention, propylene is used in a concentration of about 8 wt% to about 20 wt%, based on the weight of the total composition.

In the broadest terms of the present invention, "peroxide compound" refers to any molecule containing one or more peroxide (-O-O-) functionalities, including organic or inorganic peroxides, peroxide adducts, or peracids. These include, for example, but are not limited to, hydrogen peroxide, urea-hydrogen peroxide adduct, peracetic acid and mixtures thereof.

One or more peroxides known from the prior art which are suitable for the reaction of the propylene can be used in the present invention. Examples of the peroxides may include tert-butyl hydroperoxide and ethylbenzene hydroperoxide. In the present invention process, preference is given to using hydrogen peroxide as the peroxide compound. The present invention as described herein, therefore, also provides a process for using hydrogen peroxide as the peroxide compound. Here, preference is given to using an aqueous hydrogen peroxide.

The concentration of the hydrogen peroxide is generally between about 1% to about 35 wt%, preferably between about 1 wt% to about 20 wt%, more preferably between about 1 wt% to about 10 wt%, and most preferably between about 1 wt% to about 7 wt%, based on the weight of the total composition.

In one preferred embodiment of the present invention, an aqueous solution of hydrogen peroxide at about 30 wt% may be used such that the total amount of molecular hydrogen peroxide may be from about 1 wt% to about 7 wt%, based on the weight of the total composition.

The reaction procedure of the present invention in which the reaction of the propylene with the hydroperoxide occurs under the pressure and temperature conditions indicated herein, is preferably carried out in the presence of a suitable catalyst.

In the broadest terms of the present invention, "catalyst" can be any homogeneous or heterogeneous catalyst appropriate for the epoxidation of propylene. The catalyst may include, but are not limited to, soluble metal catalysts such as ligand-bound rhenium, tungsten, and manganese, and heterogenized forms of these, as well as solid silicate catalysts that preferably contain titanium. These solid catalysts may have the crystal structure of ZSM-5, MCM-22, MCM-41, beta-zeolites, or amorphous titanium on silica.

Preference is given to a heterogeneous catalyst and particularly to a heterogeneous catalyst which comprises a porous oxide material such as zeolite. In general, the catalysts may include, but are not limited to, a titanium-, vanadium-, chromium-, niobium- or zirconium-containing zeolite as the porous oxide material.

Specific examples of suitable zeolites are titanium-, vanadium-, chromium-, niobium-and zirconium-containing zeolites having a pentasil zeolite structure, in particular the types assigned X-ray-crystallographically to the BEA, MOR, TON, MTW, FER, MFI, MEL, CHA, ERI, RHO, GIS, BOG, NON, EMT, HEU, KFI, FAU, DDR, MTT, RUT, RTH, LTL, MAZ, GME, NES, OFF, SGT, EUO, MFS, MWW or mixed MFI/MEL structures and also ITQ-4. It is also possible to use titanium-containing zeolites having the UTD-1, CIT-1 or CIT-5 structure in the process of the present invention. Further titanium-containing zeolites which might be mentioned are those having the ZSM-48 or ZSM-12 structure. Particular preference is given to using Ti zeolites having an MFI, MEL or mixed MFI/MEL structure in the process of the present invention. Further preference is given, specifically, to the, Ti-containing zeolite catalysts which are generally designated as "TS-1", "TS-2" and "TS-3", and also Ti zeolites having a skeletal structure isomorphous with MWW-zeolite.

Particular preference is given to using a heterogeneous catalyst comprising the titanium-containing silicalite TS-1 in the process of the present invention.

It is possible to use the porous oxidic material itself as catalyst in the process of the present invention. However, it is of course also possible to use a shaped body comprising the porous oxidic material as catalyst. The shaped body from the porous oxidic material may be produced using methods well known in the prior art.

The concentration of the catalyst is generally between about 0.1 wt% to about 30 wt%, preferably between about 0.1 wt% to about 20 wt%, more preferably between about 0.1 wt% to about 10 wt%, and most preferably between about 0.5 wt% to about 5 wt%, based on the weight of the total composition.

In a preferred embodiment of the present invention, propylene is converted to propylene oxide using hydrogen peroxide and a catalyst such as titanium silicalite with MFI structure (TS-1). In this embodiment the concentration of catalyst is most preferably between about 0.5 wt% to about 3 wt%, based on the weight of the total composition.

The catalyst is typically in solid form in the reaction mixture, while the reaction is carried out in the presence of two liquids, in general an organic phase and an aqueous phase. "Bisphasic" herein means at least two liquid phases. The solid form of the catalyst can be powders for slurry type reactors or extrudates for fixed bed reactors. In one embodiment of the present invention for a slurry reactor system, the catalyst may be mixed with an organic phase, and then the aqueous phase may be added to the mixture to prevent the decomposition of the peroxide. For the slurry reactor system, for example, the size of catalyst may be less than about 100 microns. The amounts of the individual components for the embodiment should be selected based on their physical properties such that when mixed together the liquid composition comprises at least two phases, organic and aqueous.

Component (d) of the present invention is a solvent mixture; wherein the solvent mixture comprises at least (i) at least one alcohol or a combination of two or more alcohols at a predetermined concentration and (ii) at least one non-reactive co-solvent other than the solvent component (i) at a predetermined concentration as defined in claim 1, wherein the non-reactive co-solvent has a different boiling point than the propylene oxide. The solvent mixture is selected to include at least one solvent having properties such that the propylene oxide partitions into the at least one solvent present in the solvent mixture during the reaction. The at least one solvent is said to have a high affinity for the propylene oxide.

"Partitions" herein refers to the tendency of the propylene oxide to be more soluble in the solvent mixture phase than in the other phase or phases present in the reaction mixture in a reactor. It is quantified by the ratio of the propylene oxide concentration in the solvent mixture phase to the total amount of propylene oxide in the reaction mixture. Generally, the solvent is selected so that 90% or more of the propylene oxide in the reaction mixture resides in the solvent phase. Preferably, more than 99% of the propylene oxide resides in the solvent phase. Most preferably, 99.9% or more of the propylene oxide resides in the solvent phase.

In the broadest scope of the present invention, any alcohol or a mixture of two or more alcohols can be used as the first solvent component of the solvent mixture. The alcohols may include, for example, lower alcohols such as alcohols having less than 6 carbon atoms, for example methanol, ethanol, propanols (e.g. isopropanol), butanols (e.g. tert-butanol) and pentanols and a combination of two or more of these alcohols; halogenated alcohols; and mixtures thereof. Preference is given to using a C1-C4 alcohol (or mixtures thereof), and more specifically to using methanol as the alcohol for the first solvent component. In particular, methanol not only acts as a solvent, but also acts as an activator for the catalyst.

The concentration of the alcohol(s) is between 3 wt% to 10 wt%, and most preferably between about 3 wt% to about 7 wt%, based on the weight of the total composition. In a preferred embodiment of the present invention, methanol may be used at concentrations from about 3 wt% to about 7 wt%, based on the weight of the total composition.

In the broadest terms of the present invention, a "non-reacting or nonreactive co-solvent" is defined as any reagent which is inert to the reaction, i.e. the solvent does not take part in the reaction under the reaction conditions, does not react appreciably with the peroxide compound or the product under reaction conditions, is minimally soluble in water, and has a boiling point substantially different than propylene oxide. The criteria of the nonreactive co-solvent having to be "non-reactive" in order to be used in the present invention, may exclude certain reactive compounds such as for example certain olefins, carboxylates, ethers, esters, acyl halides, aldehydes, carbonates, activated aromatics, thiols, amines, molecules containing a nitro- functionality, and certain ketones which are reactive with peroxide, such as acetone and methyl ethyl ketone, for example.

Still, the co-solvent useful in the present invention, may include, inter alia, water; aliphatic, cycloaliphatic, and aromatic hydrocarbons; esters, for example methyl acetate or butyrolactone; ethers, for example diethyl ether, tetrahydrofuran, dioxane, 1,2-diethoxyethane and 2-methoxyethanol; amides, for example dimethylformamide, dimethylacetamide and N-methylpyrrolidone; sulfoxides; certain ketones; diols or polyols, preferably those having less than 6 carbon atoms; and alcohols other than or different from the first solvent component (d)(i) above. In addition, the one or more suitable solvents which are applicable in the present invention include linear and cyclic alkanes of C₃-C₁₈, halogenated hydrocarbons, deactivated aromatics, and solvents containing nitriles, for example acetonitrile; or mixtures thereof. For example, the co-solvent may include but are not limited to, carbon tetrachloride, propyl chloride, chloroform, dichloromethane, dichloroethane, hexane, octane, decalin, perfluorodecalin, mono- or poly-chlorinated benzenes, mono- or poly-brominated benzenes, acetophenone, benzonitrile, acetonitrile, trichlorotrifluoroethane, trichloroethanol, trifluoroethanol, tricresyl phosphate or mixtures of two or more of the above-mentioned compounds.

In a particularly advantageous embodiment of the present invention, the non-reacting co-solvent may be selected from those which have solubility parameters similar to propylene oxide, as estimated using Hansen parameters and a Teas plot.

The concentration of the co-solvent is between 5 wt% to 70 wt%, more preferably between about 40 wt% to about 70 wt%, and most preferably between about 50 wt% to about 70 wt%, based on the weight of the total composition.

In a preferred embodiment of the present invention, 1, 2-dichlorobenzene may be advantageously used as the non-reacting co-solvent in concentrations between about 55 wt% to about 65 wt%, based on the weight of the total composition.

Other optional components, that may be useful in the present invention, are components normally used in formulations known to those skilled in the art. For example, the optional components may comprise compounds that can be added to the composition to enhance the reaction rate, the selectivity of the reaction, and/or the catalyst lifetime. The preferred optional components and their relative concentrations useful in the composition of the present invention can be determined by the skilled artisan.

As an illustration of one preferred embodiment of the process according to the present invention, propylene oxide is prepared from propylene and hydrogen peroxide in at least one reaction step in the presence of a mixture of two or more solvents, wherein one of the solvents is methanol. One embodiment of the present invention may be directed to a specific mixture of a small amount (e.g. 3 wt % -7 wt %) of methanol along with a non-reacting co-solvent, with an excess of propylene, for example from about 8 wt% to 20 wt%, based on the weight of the total composition. The resulting reaction mixture consists of at least two liquid phases, the solid catalyst, and a vapor phase which is in contact with the other phases and components present in the reaction mixture, which increases the selectivity of the reaction without the need for other additives.

The reaction of propylene with hydrogen peroxide for the preparation of propylene oxide can be carried out herein by any suitable method, such as for example, in a batch process or continuously. In one embodiment, the process above may be carried out either in at least one batch reactor or at least one continuous reactor, or any combination of these.

With respect to the continuous processes, all suitable reactor arrangements may be useful in the present invention. Thus, for example, the propylene oxide can be prepared in a cascade of two or more reactors connected to one another in series. Conceivable processes useful in the present invention also include for example those in which reactors are arranged in parallel. Combinations of these processes are also possible. In the case where two or more reactors are connected in series, suitable intermediate treatments can also be provided between the reactors.

For example, in a first embodiment of the process according to the present invention, the propylene, the peroxide, the alcohol(s), the at least one solvent, and the catalyst are fed into at least one reactor, and the resulting liquid phases are removed from the reactor; the catalyst phase in the reactor is separated from the liquid portions of the effluents before the liquid phases are separated. This operation may be carried out in a batch process or continuously. In this first embodiment, any portion of the reactor effluent may be recycled as a feed to the reactor.

In a second embodiment of the process according to the present invention, the propylene, the peroxide, the alcohol(s), the at least one solvent, and the catalyst are fed into at least one reactor, and all of the resulting liquid phases and the catalyst are removed from the reactor and further separated in subsequent operations. This may be carried out in a batch process or continuously. In this second embodiment, any portion of the reactor effluent may be recycled as a feed to the reactor.

In a third embodiment of the process according to the present invention, at least two reactors are connected in series. The propylene, the peroxide, the alcohol(s), the at least one solvent are fed into a first reactor containing catalyst, and the liquid phases are removed from the reactor; the catalyst phase in each reactor is separated from the liquid portions of the effluents before the liquid phases are separated. In this third embodiment, all or a portion of the liquid effluent from the first reactor and subsequent reactor(s) may be fed into a subsequent reactor or reactors containing catalyst, and additionally, fresh propylene , peroxide, alcohol(s), at least one solvent, and catalyst feeds may optionally be added to each subsequent reactor along with the portions of effluent from any previous reactor.

In a fourth embodiment of the process according to the present invention, at least two reactors are connected in series. The propylene, the peroxide, the alcohol(s), and the at least one solvent are fed into the first reactor containing catalyst, and the resulting liquid phases and the catalyst are removed from the first reactor. The catalyst and the liquid phases are separated in subsequent operations. In this fourth embodiment, all or a portion of the liquid effluent and optionally a portion of the catalyst from the first reactor and subsequent reactor(s) may be fed into a subsequent reactor or reactors containing catalyst, and additionally, fresh propylene, peroxide, alcohol(s), at least one solvent, and the catalyst feeds may optionally be added to each subsequent reactor along with the portions of effluent from any previous reactor.

In a fifth embodiment of the process according to the present invention, two or more reactors are operated in parallel. The propylene, the peroxide, the alcohol(s), and the at least one solvent are fed into each reactor containing catalyst, and the resulting liquid phases are removed from each reactor. In this embodiment of the present invention, the catalyst phase in each reactor is separated from the liquid portions of the effluents before the liquid phases are separated. In this fifth embodiment of the invention a portion of the effluent stream from a reactor may be recycled back to that same reactor along with fresh feeds. The reactor effluents may remain separate or may be combined for further processing.

In a sixth embodiment of the process according to the present invention, one or more reactors are operated in parallel. The propylene, the peroxide, the alcohol(s), and the at least one solvent are fed into each reactor containing catalyst, and the resulting liquid phases and the catalyst are removed from each reactor. In this embodiment, a portion of the liquid effluent and, optionally, a portion of the catalyst from each reactor may be recycled back to that same reactor along with fresh feeds. In this sixth embodiment, the reactor effluents may remain separate or may be combined for further processing.

In a seventh embodiment of the process according to the present invention, at least two reactors are connected in series and the propylene and peroxide are fed through the reactors in counter-current flow. The olefin, none or at least a portion of the alcohol(s), the at least one solvent, and the catalyst are fed into the first reactor containing catalyst, and the peroxide and none or at least a portion of the alcohol(s) are fed into the second reactor containing catalyst or, if more than two reactors, into the final reactor. The aqueous phase of the effluent of the final reactor and any reactor downstream of the first reactor, containing partially converted peroxide, is separated from both the catalyst phase and the organic phase containing the oxirane, the at least one solvent, and any remaining propylene, and is fed to one or more upstream reactors in the series. The organic phase of the effluent from the first and subsequent reactor(s), containing any unreacted propylene, alcohol(s), at least one solvent, and oxirane, is separated from both the catalyst phase and the aqueous phase of the effluent and is fed to one or more downstream reactors containing catalyst in the series. This flow pattern repeats for all reactors in the series. In this seventh embodiment of the present invention, the catalyst phase in each reactor is separated from the liquid portions of the effluents before the liquid phases are separated.

In an eighth embodiment of the process according to the present invention, at least two reactors are connected in series and the propylene and peroxide are fed through the reactors in counter-current flow. The propylene, all or a portion of the alcohol(s), the at least one solvent, and the catalyst are fed into the first reactor containing catalyst, and the peroxide and all or a portion of the alcohol(s) are fed into the second reactor or, if more than two reactors, into the final reactor. The aqueous phase of the effluent of the final reactor and any reactor downstream of the first reactor, containing partially converted peroxide, is separated from both the catalyst phase and the organic phase containing the oxirane, the at least one solvent, and any remaining propylene, and is fed to one or more upstream reactors containing catalyst in the series. The organic phase of the effluent from the first and subsequent reactor(s), containing any unreacted propylene, alcohol(s), at least one solvent, and oxirane, is separated from both the catalyst phase and the aqueous phase of the effluent and is fed to one or more downstream reactors containing catalyst in the series. This flow pattern repeats for all reactors in the series. In this eighth embodiment of the present invention, the catalyst phase in each reactor is separated from the liquid portions of the effluents in one or more separate operation(s).

In a ninth embodiment of the process according to the present invention, at least two reactors are connected in series and the propylene and peroxide are fed through the reactors in cross-current flow. The catalyst, the first portion of propylene, a portion of the alcohol(s), the at least one solvent, and all of the peroxide are introduced into a first reactor containing catalyst, an epoxidation of the first portion of the propylene is carried out therein in order to form a first portion of the oxirane. The liquid phases of the effluent of this and subsequent reactor(s) are first separated from the catalyst, and the organic phase of the first reactor containing unreacted propylene, alcohol(s), at least one solvent, and oxirane generated is fed into at least one subsequent reactor containing catalyst, to which fresh peroxide feed is introduced. This flow pattern is repeated for all reactors in the series. The catalyst is separated from the final reactor effluent before the two liquid phases are separated.

In a tenth embodiment of the process according to the present invention, at least two reactors are connected in series and the propylene and peroxide are fed through the reactors in cross-current flow. The first portion of propylene, a portion of the alcohol(s), the at least one solvent, and all of the peroxide are introduced into a first reactor containing catalyst, an epoxidation of the first portion of the propylene is carried out therein in order to form a first portion of the oxirane. The liquid phases of the effluent of this reactor are discharged along with the catalyst, and catalyst is separated from the liquid phases in subsequent operations. The organic phase of the first and subsequent reactor(s) containing unreacted propylene, alcohol(s), at least one solvent, and oxirane generated is fed into at least one subsequent reactor containing catalyst, to which fresh peroxide feed and catalyst are introduced. This flow pattern is repeated for all reactor(s) in the series. The catalyst is separated from the final reactor effluent liquid phases in subsequent operations.

In an eleventh embodiment of the process according to the present invention, at least two reactors are connected in series and the propylene and peroxide are fed through the reactors in cross-current flow. The first portion of propylene, a portion of the alcohol(s), the at least one solvent, and all of the peroxide are introduced into a first reactor containing catalyst, an epoxidation of the first portion of the propylene is carried out therein in order to form a first portion of the oxirane. The liquid phases of the effluent of this reactor are first separated from the catalyst, and the aqueous phase of the first and subsequent reactor(s) containing unreacted peroxide is fed into at least one subsequent reactor containing catalyst, to which fresh olefin, alcohol(s), at least one solvent and catalyst are introduced. This flow pattern is repeated for all reactors in the series. The catalyst is separated from the final reactor effluent before the two liquid phases are separated.

In a twelfth embodiment of the process according to the present invention, at least two reactors are connected in series and the propylene and peroxide are fed through the reactors in cross-current flow. The first portion of propylene, a portion of the alcohol(s), the at least one solvent, and all of the peroxide are introduced into a first reactor containing catalyst, an epoxidation of the first portion of the propylene is carried out therein in order to form a first portion of the oxirane. The liquid phases of the effluent of this reactor are discharged along with the catalyst, and catalyst is separated from the liquid phases in subsequent operations. The aqueous phase of the first and subsequent reactor(s) containing unreacted peroxide is fed into at least one subsequent reactor containing catalyst, to which fresh propylene, alcohol(s), at least one solvent and catalyst are introduced. The catalyst is separated from the final reactor effluent liquid phases in subsequent operations.

The temperature and pressure of the reaction medium can either both or independently be modified during the process in the course of the preparation of propylene oxide from propylene and hydrogen peroxide.

Regarding the physical parameters, such as temperature or pressure, there are no particular restrictions. The process of the present invention may be carried out at a reaction at a temperature effective to achieve the desired reaction. The temperature of the reaction may generally be in the range of from about 10 °C to about 100 °C; more preferably from about 20 °C to about 80 °C; and most preferably from about 40 °C to about 65 °C. The other conditions of running the process of the present invention, such as pressure would be the relative pressure and other conditions of the reaction associated with the reactor composition at the particular temperature. However, the reaction is preferably carried out at pressures in the range from 1 bar to 30 bars, preferably from 8 bars to 20 bar and particularly preferably from 8 bars to 15 bars.

In one embodiment, the process of the present invention produces a waste stream with no significant amount of sodium chloride (NaCl). By "no significant amount" with reference to the sodium chloride it is meant herein as generally less than about 1 wt%, preferably less than about 0.5 wt%, and most preferably less than about 0.1%, based on the weight of the total composition.

In another embodiment of the process of the present invention, the two liquid phases formed during the reaction in the reaction mixture are separated from each other and from the catalyst. Each liquid phase is then separated into smaller components for recycle, product recovery, or purge stream isolation. Unreacted propylene, alcohol(s), and at least one non-reactive solvent are returned to the reaction section. Water and byproducts are purged from the process.

In still another embodiment, the process of the present invention may include immediately separating the water from the reaction product to minimize the contact of these compounds in the high temperature sections of the separation section and thus minimize the formation of by-products.

In another embodiment, the reaction environment may include a vapor phase in contact with the multiple liquid phase composition. Following the reaction, an organic phase and an aqueous phase are separated from the catalyst and each other. Unreacted propylene, alcohol(s), and at least one non-reactive solvent are returned to the reaction mixture; and water and byproducts are purged from the process.

In a particularly preferred embodiment, the present invention relates to a process for preparing propylene oxide comprising the steps of:
(a) in at least one reaction step, reacting propylene, a catalyst, a peroxide compound, a solvent mixture comprising at least one alcohol and at least one nonreactive solvent to form a reaction mixture as defined in claim 10, wherein the reaction mixture contents comprises at least two liquid phases and a catalyst; and simultaneously decanting the reaction mixture contents; wherein the liquid phases are separated from the catalyst; and wherein the liquid phases are recovered for further processing;
(b) separating the two liquid phases, formed in step (a), from each other forming an aqueous phase and an organic phase;
(c) separating, in at least one separation unit operation, organic compounds present in the aqueous phase of step (b), from the aqueous phase to form an organic compounds stream and a wastewater stream;
(d) recycling the organic compounds stream of step (c) to the reaction mixture; and recovering or sending the wastewater stream of step (c) to a subsequent processing operation;
(e) recovering, in at least one operation unit, the organic phase of step (b) comprising the nonreactive solvents, unreacted propylene, and propylene oxide;
(f) separating propylene oxide from the other components of the organic phase;
(g) recovering propylene oxide product from step (f);
(h) recycling the unreacted propylene and the nonreactive solvents stream of step (f) to the reaction mixture; and
(i) optionally, purging any undesired compounds which build up in the recycle streams.

Step (a) of the above process may be referred to as a "reaction/decanting step."

In the process of the present invention, the reaction of propylene with hydrogen peroxide takes place in a reactor which is suitable for this purpose. The preferred starting materials used for the reaction are propylene, hydrogen peroxide and methanol. In this process, the starting materials can be fed into the reactor in one or more streams. The streams are fed in liquid form into the reactor to form a multiphasic or at least a biphasic system. In the process of the present invention, preference is given to feeding streams consisting of the combination of the aqueous starting materials and streams consisting of the combination of the organic starting materials into the reactor.

It is possible to use any conceivable reactor known in the art which is best suited for carrying out the respective reaction. In the present invention, the term "reactor" or "reaction vessel" is not restricted to a single vessel. Rather, it is also possible to use a cascade of stirred vessels as the reactor such as described above.

The reaction vessel may comprise any well-known suitable type, including for example, one or more continuous stirred tank reactors (CSTRs) or tubular reactors; or combinations thereof. The reaction vessel may also be a batch reactor. The reactor may comprise any other well known liquid-liquid contactor, such as for example a Karr Column. For the reaction step, sufficient mixing may be required to ensure that the propylene, hydrogen peroxide and catalyst come into intimate contact. The mixing may comprise any known means for mixing such as for example, but are not limited to, stirring with an agitator, by controlling droplet size in a countercurrent extractor, by inducing shear in with a mixing element in a tubular reactor or loop reactor, or by other means. Mixing intensity should be such that the power/volume input to the reactor is preferably 1 to 100 hp/1000 gal, more preferably 1 to 10 hp/1000 gal and most preferably 2 to 4 hp/1000 gal. Interfacial area needs to be sufficiently high enough to allow sufficient mass transfer to occur for reaction to occur.

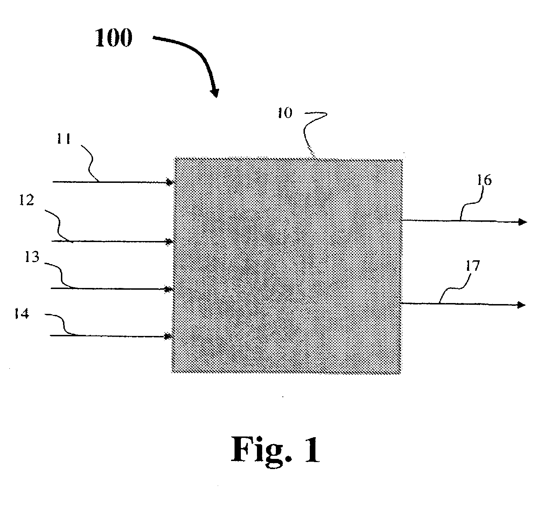

Once the catalyst is separated from the liquid reaction phases, the multiple reaction phases facilitate the subsequent recovery of propylene oxide by allowing a simple separation of the aqueous and organic phases by, for example, decantation, prior to recovery of recyclable and useful product components from each stream. See for example Figures 1-4. By reducing the amount of methanol to a predetermined level which is lower than the levels used in the prior art processes, the selectivity of the reaction to propylene oxide may be increased because the reaction of methanol with propylene oxide is reduced. When the methanol is reduced, a reaction mixture of at least two liquid phases results, which is advantageous because the majority of the propylene oxide remains in the organic phase, which reduces byproducts, for example propandiol and methoxypropanols, formed from the reaction of propylene oxide with water. Also, many of the heavy byproducts formed remain in the aqueous phase. It is a critical element of the reaction that the reaction remains in at least two liquid phases so that the propylene oxide formed will be sequestered away from the aqueous phase and into one of the organic phases. This greatly reduces the hydrolysis of the propylene oxide to by-products.

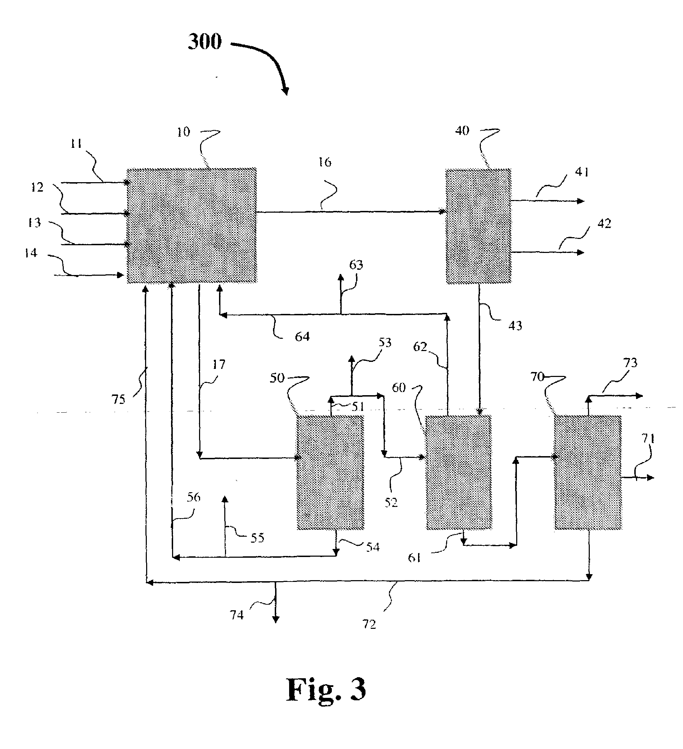

The reaction may be carried out in a mixed solvent system consisting of a small amount of methanol with a non-reacting co-solvent. The advantages that the system of the present invention offers are increased catalyst lifetime and decreased energy costs for separation. For example, based on a simulation of the process taught by U.S. Patent No. 7,138,534 the embodiment presented as Figure 3 results in a reduction of at least 50% in energy required per pound of propylene oxide produced. The presence of methanol in the reactor at a concentration of 3% to 10%, and most preferably between about 3% to about 7%, based on the weight of the total composition, extends catalyst lifetime as described in the examples.

Another advantage of the present invention is that the addition of the non-reacting co-solvent increases the catalyst lifetime by reducing the plugging of the catalyst pores.

Still another advantage of the present invention is that the biphasic nature of the reaction mixture allows the separation of the organic and aqueous phases by decanting, which reduces the size of distillation towers and the steam consumption, compared to a process that would use a high level of methanol.

It has been discovered that TS-1 activity in the epoxidation of propylene with H₂O₂ can be maintained by using a mixture of solvents comprising less than 10% methanol along with a non-reactive co-solvent.

The present invention will now be described with reference to the non-limiting embodiments shown in Figures 1-4. In Figures 1-4 illustrating the process of the present invention, like reference numerals are used to denote like parts throughout the several Figures. In the following descriptions the term "vessel" is defined herein to mean one or more pieces of equipment. "Reaction/decantation vessel" is defined herein to mean any of the embodiments previously described above.

Figure 1, for example, shows a process of the present invention, generally indicated by the numeral 100, comprising a reaction/decantation vessel 10, wherein a propylene reactant feed stream 11 may be introduced into the reaction/decantation vessel 10. Figure 1 represent the reaction/decantation steps in the first, third, fifth, seventh, ninth and eleventh embodiments described above. The reaction/decantation vessel 10 may comprise any well-known suitable type, including for example, one or more continuous stirred tank reactors (CSTRs) or tubular reactors; or combinations thereof such as for example as described above. The reaction vessel 10 may also comprise a batch reactor.

Also introduced into vessel 10, are a hydroperoxide feed stream 12, such as hydrogen peroxide; a single or mixed alcohols feed stream 13 containing a minimum amount of alcohol such as methanol; and an inert solvent feed stream 14 comprising, for example, ortho dichlorobenzene. The reaction/decantation vessel 10 contains a solid catalyst, for example a titanium silicalite such as TS-1 which remains within the boundaries of the equipment as shown in Figure 1. Streams 11, 12, 13, and 14 may be introduced into vessel 10 either separately or together. In addition, optionally, all of the streams 11, 12, 13, and 14 may be combined together into one feed stream. Any of the streams 11, 12, 13, or 14 may be introduced at a single point or at multiple points of vessel 10. The relative amounts of streams 11, 12, 13, and 14 are chosen so that when they are combined in vessel 10 a separate aqueous phase exists along with one or more organic phases, the solid catalyst phase, and optionally a vapor phase above the reaction liquids and catalyst.

In vessel 10, propylene may be partially or fully converted to propylene oxide with the TS-1 catalyst plus reaction products of propylene oxide and methanol. Stream 16 containing for example, primarily water, unreacted hydrogen peroxide, propanediol, methoxypropanol, and mixed alcohols may be removed from vessel 10; and my be sent to storage, to another operation for further processing such as purification, or to other equipment for further reaction or processing. Stream 17 containing for example, primarily unreacted propylene, propylene oxide, a single or mixed alcohols, and nonreactive solvent such as ortho dichlorobenzene may be removed from vessel 10; and may be sent to storage, to another operation for further processing such as purification, or to other equipment for further reaction or processing.

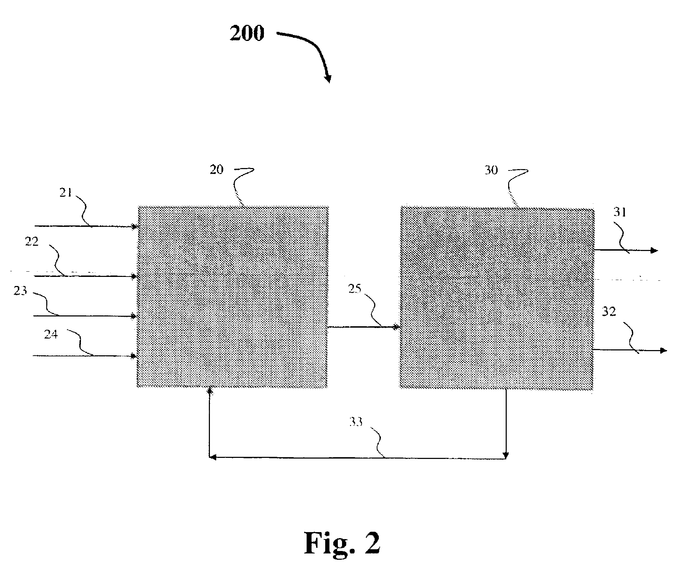

Figure 2 shows another embodiment of the process of the present invention generally indicated by numeral 200, wherein a propylene feed stream, 21, may be introduced into a reaction vessel, 20. Figure 2 represents the reaction/decantation steps in the second, fourth, sixth, eighth, tenth, and twelfth embodiments described above. The reaction vessel 20 may comprise any well-known suitable type, including for example, one or more continuous stirred tank reactors (CSTRs) or tubular reactors; or combinations thereof such as for example as described above. It may also be a batch reactor. Also fed to vessel 20, are a hydroperoxide feed stream 22, such as hydrogen peroxide; a single or mixed alcohols feed stream 23 containing a minimum amount of alcohol such as methanol; and an inert solvent feed stream 24 comprising, for example, ortho dichlorobenzene. The reaction vessel 20 contains a solid catalyst 25, for example a titanium silicalite such as TS-1.

Streams 21, 22, 23, and 24, may be introduced into vessel 20 either separately or together. In addition, optionally, all of the streams 21, 22, 23 and 24 may be combined together into one feed stream. Any of the streams 21, 22, 23 or 24 may be introduced at a single point or at multiple points of vessel 20. The relative amounts of streams 21, 22, 23, and 24 are chosen so that when they are combined in vessel 20 a separate aqueous phase exists along with one or more organic phases, the solid catalyst phase, and optionally a vapor phase above the reaction liquids and catalyst.

Also fed to vessel 20 may be a recycle stream 33 comprising mostly solid catalyst, TS-1. Optionally, the solid catalyst may remain in vessel 20 in which case stream 33 would have no flow.

In the embodiment of the current invention shown in Figure 2, the mixed contents of vessel 20 containing all liquid phases and optionally the solid catalyst may be fed to vessel 30 as stream 26. Vessel 30 may comprise any well-known suitable separation vessel, including decantation, hydrocyclone, mechanically driven high gravity devices, or any suitable known separation apparati known in the art. Stream 31, consisting for example, primarily of water, unreacted hydrogen peroxide, propanediol, methoxypropanol, and single or mixed alcohols may be removed from vessel 30 and my be sent to storage, for further processing such as purification, or to other equipment for further reaction. Stream 32, containing for example, primarily unreacted propylene, propylene oxide, single or mixed alcohols, and nonreactive solvent such as ortho dichlorobenzene may be removed from vessel 30 and may be sent to storage, to another operation for further processing such as purification, or to other equipment for further reaction or processing.

Figure 3 shows another embodiment of the process of the present invention generally indicated by numeral 300, wherein a propylene feed stream 11 may be introduced into a reaction/decantation vessel 10. The reaction/decantation vessel 10 may comprise any well-known suitable vessel type, including for example, one or more continuous stirred tank reactors (CSTRs) or tubular reactors; or combinations thereof. The vessel 10 may also be a batch reactor. The liquid phases and catalyst may be separated by any well known means of separation such as, for example, decantation, hydrocyclone, mechanically driven high gravity devices, any suitable known separation apparati, or any combination thereof.

Also introduced into vessel 10, are a hydroperoxide feed stream, 12, such as hydrogen peroxide; a single or mixed alcohols feed stream 13 containing a minimum amount of alcohol such as methanol; and an inert solvent feed stream 14 comprising, for example, ortho dichlorobenzene. The reaction/decantation vessel, 10, contains a solid catalyst, for example a titanium silicalite such as TS-1 which remains within the boundaries of the equipment as shown in Figure 3 as vessel 10. Streams 11, 12, 13, and 14 may be introduced into vessel 10 either separately or together. In addition, optionally, all of the streams 11, 12, 13, and 14 may be combined together into one feed stream. Any of the streams 11, 12, 13, or 14 may be introduced at a single point or at multiple points of vessel 10. The relative amounts of streams 11, 12, 13, and 14 are chosen so that when they are combined in vessel 10 a separate aqueous phase exists along with one or more organic phases, the solid catalyst phase, and optionally a vapor phase above the reaction liquids and catalyst.

In vessel 10, propylene may be partially or fully converted to propylene oxide with the TS-1 catalyst, plus reaction products of propylene oxide and methanol, and propanediol from the hydrolysis of propylene oxide. Stream 16 containing for example, primarily water, unreacted hydrogen peroxide, propanediol, methoxypropanol and single or mixed alcohols may be removed from vessel 10 and sent to vessel 40. Vessel 40 produces stream 42 composed for example, primarily of water, propanediol, methoxypropanol and stream 41 composed for example primarily of light feed impurities or by products. Both stream 41 and stream 42 are purged from the process. A stream from vessel 40 may be sent to vessel 60 as stream 43 for further recovery of unreacted propylene, single or mixed alcohols and propylene oxide.

Stream 17 containing for example, primarily unreacted propylene, propylene oxide, single or mixed alcohols, and nonreactive solvent such as ortho dichlorobenzene may be removed from vessel 10 and may be sent to vessel 50. Vessel 40 may comprise, for example, a distillation column or a combination of two or more distillation columns.

In vessel 50 the non-reactive solvent, for example ortho dichlorobenzene, may be separated and exits vessel 50 as stream 54. Optionally, a portion of stream 54 may be purged from the process as stream 55. Stream 56 as the net of stream 54 less any material removed as stream 55 may be recycled to vessel 10. Stream 51, comprising for example primarily unreacted propylene, mixed alcohols, and propylene oxide, exits vessel 50. Optionally, a portion of stream 51 may be removed and purged from the process as stream 53. Stream 52 as the net of stream 51 less any material removed as stream 53 may be fed to vessel 60. Vessel 50 may comprise, for example, a distillation column or a combination of two or more distillation columns.

In vessel 60, the unreacted propylene may be separated and removed from vessel 60 as stream 62. Optionally, a portion of stream 62 may be purged from the process as stream 63. The net of stream 62 less any material removed as stream 63 may be recycled back to vessel 10 as stream 64. Stream 61, comprising for example propylene oxide, and compounds with a higher boiling point than propylene oxide, may be fed to vessel 70. Vessel 60 may comprise, for example, a distillation column or a combination of two or more distillation columns.

In vessel 70 the product propylene oxide may be separated and produced as stream 71. Compounds with boiling points lower than propylene oxide are discharged from vessel 70 as stream 73. Compounds with boiling points higher than propylene oxide are discharged from vessel 70 as stream 72. Optionally, a portion of stream 72 may be purged from the process as stream 74. The net of stream 72 less any material removed as stream 74 may be recycled back to vessel 10 as stream 75. Vessel 70 may comprise, for example, a distillation column or a combination of two or more distillation columns.

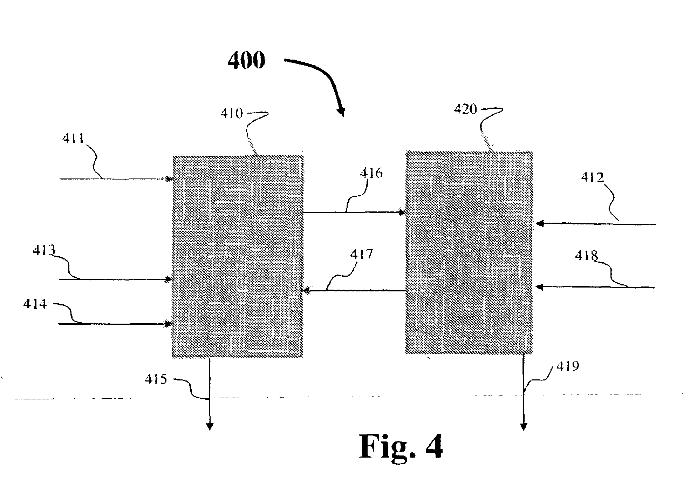

Figure 4, shows another embodiment of the process of the present invention, generally indicated by the number 400, comprising two reactors, vessels 410 and 411 connected in series as described above with reference to the seventh embodiment. Feeds are introduced to vessel 410 as a propylene feed stream 411, a single or mixed alcohol stream 413, and an inert solvent feed stream 414. Streams 411, 413 and 414 may be introduced into vessel 410 either separately or together. In addition, optionally, all of the streams 411, 413, and 414 may be combined together into one feed stream. Any of the streams 411, 413, or 414 may be introduced at a single point or at multiple points of vessel 410. Feeds are introduced to vessel 420 as a peroxide feed stream 412 and a single or mixed alcohol stream 478. Streams 412 and 478 may be introduced into vessel 420 either separately or together. In addition, optionally, all of the streams 412, 478 may be combined together into one feed stream. Any of the streams 412 and 478 may be introduced at a single point or at multiple points of vessel 420.

The aqueous effluent from vessel 420 as stream 417 is fed to vessel 410. The organic effluent from vessel 410 as stream 416 is fed to vessel 420. The organic phase effluent from vessel 420 is leaves the reactor series as stream 419. The aqueous phase effluent from vessel 410 leaves the reactor series as stream 415.

The propylene oxide produced by the process of the present invention can be used in various applications, including for example, in the production of polyether polyols for the production of polyurethane plastics or in the production of propylene glycol.

### EXAMPLES

The following example further illustrates the present invention in detail but is not to be construed to limit the scope thereof.

The following standard analytical equipments and methods are used in the Example:
Gas chromatography was performed on an HP 6890 series G1530A GC with an HP 7682 series injector and FID detection. An HP-1701 14% cyanopropyl phenyl methyl column of length 60.0 m, diameter 320.00 µm, and thickness 1.00 µm was used at temperatures from 35°C to 250 °C.

Peroxide amounts were analyzed by iodometric titration using 0.01N sodium thiosulfate. The peroxide concentration may be calculated as follows: ppm H₂O₂ = (mL titrant used) (0.01 N)(17000)/g sample. Titrations were performed using a Mettler Toledo DL5x V2.3 titrator with a DM140 sensor.

### Example 1

Propylene (100 g), TS-1 catalyst (10 g), methanol (25 g), and o-dichlorobenzene (295 g) are added to a 750-mL jacketed reactor with a stainless steel cooling coil, thermocouple, mechanical stirrer, addition funnel, N₂ purge with gas scrubber, and reflux condenser/cold finger combination. About 35 wt%/aq. hydrogen peroxide (70 g) is charged to the addition funnel, and then added to the reactor slowly after the propylene/catalyst/methanol mixture is brought to 25.5 °C. The mixture is stirred at 600 rpm, and the exothermic reaction raises the temperature of the mixture up to 40 °C, where the temperature is maintained using the cooling coil.

Samples are taken from the reactor and then analyzed by gas chromatography (GC). When the reaction is deemed complete by analyzing propylene oxide via GC (after 60 minutes), both phases are analyzed by GC and the amount of peroxide remaining is determined by titration with sodium thiosulfate. The results of this experiment are as follows:
Conversion of hydrogen peroxide: 99%.
Selectivity (of H₂O₂ reacted, that portion making propylene oxide): 95%.
Yield ( propylene oxide made versus total propylene which would have resulted if all H₂O₂ went to propylene oxide): 94%.

[H₂O₂ Conversion * Selectivity to PO = 99/100 * 94/99 = 94% Yield to PO].

## Claims

1. A multiple liquid phase composition, useful for preparing propylene oxide, comprising a reactive mixture of: (a) propylene; (b) a peroxide compound, (c) at least one catalyst, and (d) a solvent mixture; wherein the solvent mixture comprises a mixture of two or more solvents comprising (i) at least one alcohol, and (ii) at least one non-reactive co-solvent other than the at least one alcohol; wherein the at least one alcohol and the at least one non-reactive co-solvent are mixed such that the at least one alcohol is from 3 weight percent to 10 weight percent based on a weight of the total composition and the at least one non-reactive co-solvent is from 5 weight percent to 70 weight percent based on the weight of the total composition; wherein the at least one co-solvent has a different boiling point than the propylene oxide; and wherein the propylene oxide is capable of partitioning into at least one of the solvents present in the solvent mixture during the reaction.

2. The composition of claim 1, wherein the concentration of propylene comprises from 10 weight percent to 90 weight percent.

3. The composition of claim 1, wherein the concentration of the peroxide compound comprises from 1 weight percent to 35 weight percent.

4. The composition of claim 1, wherein the at least one catalyst comprises a titanium silicate catalyst.

5. The composition of claim 1, wherein the concentration of the at least one catalyst comprises from 0.1 weight percent to 10 weight percent.

6. The composition of claim 1, wherein the at least one alcohol of the solvent mixture comprises a C1 to C4 alcohol.

7. The composition of claim 1, wherein the at least one alcohol of the solvent mixture comprises methanol.

8. The composition of claim 7, wherein the concentration of the methanol comprises less than 10 weight percent.

9. The composition of claim 1, wherein the at least one non-reactive co-solvent comprises linear and cyclic alkanes of C₃-C₁₈; halogenated hydrocarbons; deactivated aromatics; amides; solvents containing nitriles; alcohols different from the alcohol solvent; or mixtures thereof

10. A process for preparing propylene oxide from propylene and a peroxide compound comprising reacting (a) propylene with (b) a peroxide compound, in the presence of (c) at least one catalyst and (d) in the presence of a solvent mixture; wherein the solvent mixture comprises at least (i) at least one alcohol and (ii) at least one non-reactive co-solvent such that the at least one alcohol is from 3 weight percent to 10 weight percent based on a total weight of (a), (b), (c), and (d) and the at least one non-reactive co-solvent is from 5 weight percent to 70 weight percent based on the total weight of (a), (b), (c), and (d); wherein the co-solvent has a different boiling point than propylene oxide; and wherein propylene oxide partitions into a high affinity solvent during the reaction.

11. The process of claim 10, wherein the process produces a waste stream with an amount of sodium chloride (NaCl) of less than 0.1 weight percent.

12. The process of claim 10 or claim 11, wherein the at least one peroxide compound comprises hydrogen peroxide; wherein the at least one catalyst comprises a titanium silicate catalyst; wherein the at least one alcohol of the solvent mixture comprises methanol; and wherein the at least one non-reactive co-solvent comprises linear or cyclic alkanes of C3-C18, halogenated hydrocarbons, deactivated aromatics, amides, solvents containing nitriles, alcohols, halogenated alcohols, or mixtures thereof.

13. The process of any of claims 10-12 wherein the reaction step is carried out in at least 2 reactors connected in series and wherein the olefin and peroxide streams are fed through the reactors in counter-current flow, in co-current flow or in cross-current flow.

14. The process of claim 13, wherein the catalyst and liquid phases are separated in a subsequent operation.

## Patentansprüche

1. Eine Mehrflüssigphasenzusammensetzung, die zum Zubereiten von Propylenoxid nützlich ist, die eine reaktionsfähige Mischung aus Folgendem beinhaltet: (a) Propylen; (b) eine Peroxidverbindung, (c) mindestens einen Katalysator und (d) eine Lösungsmittelmischung; wobei die Lösungsmittelmischung eine Mischung aus zwei oder mehr Lösungsmitteln beinhaltet, beinhaltend: (i) mindestens einen Alkohol und (ii) mindestens ein nicht reaktionsfähiges Co-Lösungsmittel, das sich von dem mindestens einen Alkohol unterscheidet; wobei der mindestens eine Alkohol und das mindestens eine nicht reaktionsfähige Co-Lösungsmittel gemischt werden, so dass der mindestens eine Alkohol von 3 Gewichtsprozent bis 10 Gewichtsprozent, in Bezug auf ein Gewicht der Gesamtzusammensetzung, beträgt und das mindestens eine nicht reaktionsfähige Co-Lösungsmittel von 5 Gewichtsprozent bis 70 Gewichtsprozent, in Bezug auf das Gewicht der Gesamtzusammensetzung, beträgt; wobei das mindestens eine Co-Lösungsmittel einen anderen Siedepunkt als das Propylenoxid aufweist; und wobei das Propylenoxid in der Lage ist, sich während der Reaktion in mindestens einem der in der Lösungsmittelmischung vorhandenen Lösungsmittel zu verteilen.

2. Zusammensetzung gemäß Anspruch 1, wobei die Konzentration von Propylen von 10 Gewichtsprozent bis 90 Gewichtsprozent beträgt.

3. Zusammensetzung gemäß Anspruch 1, wobei die Konzentration der Peroxidverbindung von 1 Gewichtsprozent bis 35 Gewichtsprozent beträgt.

4. Zusammensetzung gemäß Anspruch 1, wobei der mindestens eine Katalysator einen Titansilicatkatalysator beinhaltet.

5. Zusammensetzung gemäß Anspruch 1, wobei die Konzentration des mindestens einen Katalysators von 0,1 Gewichtsprozent bis 10 Gewichtsprozent beträgt.

6. Zusammensetzung gemäß Anspruch 1, wobei der mindestens eine Alkohol der Lösungsmittelmischung einen C1-C4-Alkohol beinhaltet.

7. Zusammensetzung gemäß Anspruch 1, wobei der mindestens eine Alkohol der Lösungsmittelmischung Methanol beinhaltet.

8. Zusammensetzung gemäß Anspruch 7, wobei die Konzentration des Methanols weniger als 10 Gewichtsprozent beträgt.

9. Zusammensetzung gemäß Anspruch 1, wobei das mindestens eine nicht reaktionsfähige Co-Lösungsmittel Folgendes beinhaltet: lineare und zyklische C₃-C₁₈-Alkane; Halogenkohlenwasserstoffe; desaktivierte Aromaten; Amide; Nitrile enthaltende Lösungsmittel; sich von dem Alkohollösungsmittel unterscheidende Alkohole; oder Mischungen davon.

10. Ein Verfahren zum Zubereiten von Propylenoxid aus Propylen und einer Peroxidverbindung, das Folgendes beinhaltet: Reagierenlassen von (a) Propylen mit (b) einer Peroxidverbindung in Gegenwart von (c) mindestens einem Katalysator und (d) in Gegenwart einer Lösungsmittelmischung; wobei die Lösungsmittelmischung mindestens Folgendes beinhaltet: (i) mindestens einen Alkohol und (ii) mindestens ein nicht reaktionsfähiges Co-Lösungsmittel, so dass der mindestens eine Alkohol von 3 Gewichtsprozent bis 10 Gewichtsprozent, in Bezug auf ein Gesamtgewicht von (a), (b), (c) und (d), beträgt und das mindestens eine nicht reaktionsfähige Co-Lösungsmittel von 5 Gewichtsprozent bis 70 Gewichtsprozent, in Bezug auf das Gesamtgewicht von (a), (b), (c) und (d), beträgt; wobei das Co-Lösungsmittel einen anderen Siedepunkt als das Propylenoxid aufweist; und wobei sich das Propylenoxid während der Reaktion in einem Hochaffinitätslösungsmittel verteilt.

11. Verfahren gemäß Anspruch 10, wobei das Verfahren einen Abfallstrom mit einer Menge an Natriumchlorid (NaCl) von weniger als 0,1 Gewichtsprozent herstellt.

12. Verfahren gemäß Anspruch 10 oder Anspruch 11, wobei die mindestens eine Peroxidverbindung Wasserstoffperoxid beinhaltet; wobei der mindestens eine Katalysator ein Titansilicatkatalysator ist; wobei der mindestens eine Alkohol der Lösungsmittelmischung Methanol beinhaltet; und wobei das mindestens eine nicht reaktionsfähige Co-Lösungsmittel lineare oder zyklische C3-C18-Alkane, Halogenkohlenwasserstoffe, desaktivierte Aromaten, Amide, Nitrile enthaltende Lösungsmittel, Alkohole, Halogenalkohole oder Mischungen davon beinhaltet.

13. Verfahren gemäß einem der Ansprüche 10-12, wobei der Reaktionsschritt in mindestens 2 in Reihe verbundenen Reaktoren durchgeführt wird und wobei die Olefin- und Peroxidströme in einem Gegenstrom, in einem Gleichstrom oder in einem Kreuzstrom durch die Reaktoren gespeist werden.

14. Verfahren gemäß Anspruch 13, wobei der Katalysator und die Flüssigphasen in einem nachfolgenden Vorgang getrennt werden.

## Revendications

1. Une composition à phases liquides multiples, utile pour préparer de l'oxyde de propylène, comprenant un mélange réagissant de : (a) propylène ; (b) un composé peroxyde, (c) au moins un catalyseur, et (d) un mélange de solvants ; dans laquelle le mélange de solvants comprend un mélange de deux solvants ou plus comprenant (i) au moins un alcool, et (ii) au moins un co-solvant non réagissant autre que cet au moins un alcool ; dans laquelle cet au moins un alcool et cet au moins un co-solvant non réagissant sont mélangés de façon à ce que cet au moins un alcool fasse de 3 pour cent en poids à 10 pour cent en poids rapporté à un poids de la composition totale et cet au moins un co-solvant non réagissant fasse de 5 pour cent en poids à 70 pour cent en poids rapporté au poids de la composition totale ; dans laquelle cet au moins un co-solvant a un point d'ébullition différent de l'oxyde de propylène ; et dans laquelle l'oxyde de propylène est capable de se répartir dans au moins un des solvants présents dans le mélange de solvants au cours de la réaction.

2. La composition de la revendication 1, dans laquelle la concentration de propylène consiste en de 10 pour cent en poids à 90 pour cent en poids.

3. La composition de la revendication 1, dans laquelle la concentration du composé peroxyde consiste en de 1 pour cent en poids à 35 pour cent en poids.

4. La composition de la revendication 1, dans laquelle cet au moins un catalyseur comprend un catalyseur silicate de titane.

5. La composition de la revendication 1, dans laquelle la concentration de cet au moins un catalyseur consiste en de 0,1 pour cent en poids à 10 pour cent en poids.

6. La composition de la revendication 1, dans laquelle cet au moins un alcool du mélange de solvants comprend un alcool en C1 à C4.

7. La composition de la revendication 1, dans laquelle cet au moins un alcool du mélange de solvants comprend du méthanol.

8. La composition de la revendication 7, dans laquelle la concentration du méthanol consiste en moins de 10 pour cent en poids.

9. La composition de la revendication 1, dans laquelle cet au moins un co-solvant non réagissant comprend des alcanes linéaires et cycliques de C₃ à C₁₈; des hydrocarbures halogénés ; des aromatiques désactivés ; des amides ; des solvants contenant des nitriles ; des alcools différents du solvant alcool ; ou des mélanges de ceux-ci.

10. Un procédé pour préparer de l'oxyde de propylène à partir de propylène et d'un composé peroxyde comprenant les étapes consistant à faire réagir (a) du propylène avec (b) un composé peroxyde, en présence de (c) au moins un catalyseur et (d) en présence d'un mélange de solvants ; dans lequel le mélange de solvants comprend au moins (i) au moins un alcool et (ii) au moins un co-solvant non réagissant de façon à ce que cet au moins un alcool fasse de 3 pour cent en poids à 10 pour cent en poids rapporté à un poids total de (a), (b), (c), et (d) et cet au moins un co-solvant non réagissant fasse de 5 pour cent en poids à 70 pour cent en poids rapporté au poids total de (a), (b), (c), et (d) ; dans lequel le co-solvant a un point d'ébullition différent de l'oxyde de propylène ; et dans lequel l'oxyde de propylène se répartit dans un solvant à haute affinité au cours de la réaction.

11. Le procédé de la revendication 10, le procédé produisant un écoulement résiduaire avec une quantité de chlorure de sodium (NaCl) inférieure à 0,1 pour cent en poids.

12. Le procédé de la revendication 10 ou la revendication 11, dans lequel cet au moins un composé peroxyde comprend du peroxyde d'hydrogène ; dans lequel cet au moins un catalyseur comprend un catalyseur silicate de titane ; dans lequel cet au moins un alcool du mélange de solvants comprend du méthanol ; et dans lequel cet au moins un co-solvant non réagissant comprend des alcanes linéaires ou cycliques de C3 à C18, des hydrocarbures halogénés, des aromatiques désactivés, des amides, des solvants contenant des nitriles, des alcools, des alcools halogénés, ou des mélanges de ceux-ci.

13. Le procédé de n'importe lesquelles des revendications 10 à 12 dans lequel l'étape de réaction est effectuée dans au moins 2 réacteurs connectés en série et dans lequel les écoulements d'oléfine et de peroxyde sont introduits dans les réacteurs en flux à contre-courant, en flux à co-courant ou en flux à courant croisé.

14. Le procédé de la revendication 13, dans lequel le catalyseur et les phases liquides sont séparés dans une opération ultérieure.
